# EUROPEAN PATENT SPECIFICATION

(11) **EP 3 818 508 B1**
(45) Date of publication and mention of the grant of the patent: **29.06.2022**
(21) Application number: 18737915.1
(22) Date of filing: 06.07.2018
(51) Int. Cl.: G08B 21/04, G01C 5/06

(54) **FALL DETECTION SYSTEM AND METHOD**
FALLDETEKTIONSSYSTEM UND -VERFAHREN
SYSTÈME ET PROCÉDÉ DE DÉTECTION DE CHUTE

(43) Date of publication of application: 12.05.2021
(73) Proprietor: PINK NECTARINE HEALTH AB, 103 11 Stockholm (SE)
(72) Inventor: WIDGREN, Anders, 103 11 Stockholm (SE); LINGHAMMAR, Tobias, 182 45 Enebyberg (SE)
(74) Representative: Keijser Bergöö, Malin Katarina
(86) International application number: PCT/EP2018/068426
(87) International publication number: WO 2019/008162

(56) References cited:
- WO-A1-2011/055255
- US-A1- 2010 052 896
- US-A1- 2016 038 061
- US-A1- 2016 253 890
- US-A1- 2017 000 387

## Description

### TECHNICAL FIELD

The present disclosure relates generally to fall detection systems and methods.

### BACKGROUND

Elderly, sick or injured people may be prone to falling accidents. Since they may not have the strength or mobility to stand up after a fall has occurred, they may need help in such situations. This could be the case even if the fall itself has not caused any injury. It may therefore be desirable to monitor such an individual so that help can be dispatched when a fall has occurred.

US 2010/0052896 describes a fall detection system in which elderly, sick or injured people may be equipped with a wristband comprising a mobile barometric pressure sensor. The pressure sensor in the wristband communicates with a reference barometric pressure sensor installed at a known height (e.g. on a wall), which provides a reference pressure signal for the known height. The system comprises a processor that, based on the reference pressure of the reference pressure sensor, determines the absolute height of the mobile pressure sensor above the floor, and may activate an alarm if it is determined that the height of the mobile pressure sensor is below a predetermined threshold height.

US 7893844 describes a fall detection system in which a resident carries a resident height detection device which can determine its height over the floor. The system may determine that a fall has occurred if the resident height detection device has remained within a predetermined threshold distance of the floor for more than a given period of time. A reference sensor may be mounted at a known height (e.g. on a wall) and provide a calibration signal to calibrate the height of the resident height detection device.

US 2015/0025817 describes a fall detection system in which elderly people carry a user device that determines a fall by comparing a height change of the user device with a threshold height change. The threshold height change can be adapted to the height at which the user wears or carries the user device.

US2016/0253890 describes a system for monitoring the use of a walker. The system comprises at least one sensor adapted for retrofitting to a walker, for measuring a physical parameter at at least one location on the walker and for sending at least one feedback signal indicating a value of the physical parameter measured.

WO2011055255 describes a system and a method for revoking a fall alarm of a user after the fall is detected. The system comprises an altimeter and an accelerator, worn by the user, that acquire a plurality of parameters relating to the fall. The processor processes the plurality of parameters to determine a posture change of the user within a first predefined period after the fall is detected. The controller revokes the fall alarm when the posture change indicates that the user has stood up.

### PROBLEMS WITH THE PRIOR ART

It is very difficult to accurately determine the absolute height of a pressure sensor above the floor. This means that systems relying on determining an absolute height will not be reliable.

Further, the movement pattern varies between individuals, and may also vary e.g. based on the layout of the house or apartment. For certain individuals in certain houses or apartments, it may e.g. be common to bend down close to the floor to e.g. fetch things from drawers which are located close to the floor. Absolute settings of threshold heights will therefore give many false alarms.

There is thus a need for an improved fall detection system.

### SUMMARY

The above described problem is addressed by the claimed fall detection system. The system comprises at least one processing device, at least one personal module comprising a personal module pressure sensor, and at least one reference module comprising a reference module pressure sensor. The at least one processing device is arranged to receive personal pressure data from the at least one personal module pressure sensor, receive reference pressure data from the at least one reference module pressure sensor, determine a personal pressure using the personal pressure data and the reference pressure data, determine an individual personal pressure profile based on historical personal pressure data for the individual, compare the personal pressure with the determined individual personal pressure profile, and set a fall detection alert based at least on whether the personal pressure lies beyond the individual personal pressure profile. Since the individual personal pressure profile is determined based on historical personal pressure data for the individual, variations in movement pattern between different individuals and between different houses or apartments will be compensated, and the number of false alarms will be reduced.

In embodiments, the individual personal pressure profile comprises an individual personal pressure threshold, and the at least one processing device is arranged to set the fall detection alert based at least on whether the personal pressure is above the individual personal pressure threshold.

In embodiments, the at least one processing device is arranged to determine the personal pressure as an absolute personal pressure calculated based on the personal pressure data and the reference pressure data, and determine the individually determined personal pressure profile based on the historical absolute personal pressure of the individual.

In embodiments, the at least one processing device is arranged to determine the individual personal pressure profile based on the probability distribution of the historical personal pressure of the individual. These are simple yet reliable ways of determining the individual personal pressure profile.

In embodiments, the personal module further comprises a movement sensor, e.g. an accelerometer, and the at least one processing device is further arranged to analyze the signal from the movement sensor and set the fall detection alert based also on this signal. A fall may comprise a characteristic movement that can be detected by a movement sensor, which means that the signal from a movement sensor can be used to avoid false alarms.

In embodiments, the processing device is further arranged to send an alarm signal based on the fall detection alert. This may call the attention of people who may help the individual who has fallen, such as relatives and/or healthcare personnel.

In embodiments, the system comprises a number of reference modules, and the at least one processing device is arranged to determine the personal pressure using reference pressure data from the reference module that is closest to the personal module. Since the reference pressure data should correspond as closely as possible to the personal pressure data, the reference pressure data should be collected as simultaneously as possible from the reference module that is closest to the personal module when the personal pressure data is collected.

The above described problem is further addressed by the claimed fall detection method. The method comprises receiving, in at least one processing device, personal pressure data from a personal module pressure sensor arranged in a personal module, receiving, in the at least one processing device, reference pressure data from a reference module pressure sensor arranged in a reference module, determining a personal pressure using the personal pressure data and the reference pressure data, determining an individual personal pressure profile, which may e.g. be an individual personal pressure threshold, based on the historical personal pressure of the individual, comparing the personal pressure with the individual personal pressure profile, and setting a fall detection alert based at least on whether the personal pressure lies beyond the individual personal pressure profile. Since the personal pressure profile is individually determined, the claimed method compensates for variations in movement pattern between different individuals and between different houses or apartments, and thus reduces the number of false alarms.

In embodiments, the individual personal pressure profile comprises an individual personal pressure threshold, and the method comprises setting the fall detection alert based at least on whether the personal pressure is above the individual personal pressure threshold.

In embodiments, the determining of the personal pressure comprises calculating the personal pressure as an absolute personal pressure based on the personal pressure data and the reference pressure data, and the determining of the individually determined personal pressure profile is based on the historical absolute personal pressure of the individual.

In embodiments, the method further comprises determining the individual personal pressure profile based on the probability distribution of historical personal pressure data for the individual. These are simple yet reliable ways of determining the individual personal pressure profile.

In embodiments, the method further comprises inputting the personal pressure into a machine learning system that has been trained using historical personal pressures representing detected falls, and the setting of the fall detection alert is based also on whether this machine learning system classifies the personal pressure as a fall. The machine learning system may have been trained using historical personal pressures representing detected falls for the individual, or for many individuals.

In embodiments, the method further comprises setting the fall detection alert based also on a signal from a movement sensor, e.g. an accelerometer, arranged in the personal module. A fall may comprise a characteristic movement that can be detected by a movement sensor, which means that the signal from a movement sensor can be used to avoid false alarms.

In embodiments, the method further comprises sending an alarm signal based on the fall detection alert. This may call the attention of people who may help the individual who has fallen, such as relatives and/or healthcare personnel.

In embodiments, the method further comprises determining the personal pressure using reference pressure data from the reference module that is closest to the personal module. Since the reference pressure data should correspond as closely as possible to the personal pressure data, the reference pressure data should be collected as simultaneously as possible from the reference module that is closest to the personal module when the personal pressure data is collected.

The at least one processing device may be a personal module processing device, a reference module processing device, or another processing device, such as a remote processing device, which may e.g. be comprised in a web server and/or be cloud based. The at least one processing device may also be a combination of any number of processing devices, so that some of the processing takes place in one processing device and some of the processing takes place in one or more other processing devices. It is thus not necessary for all of the processing to take place in the same processing device.

The scope of the invention is defined by the claims, which are incorporated into this section by reference. A more complete understanding of embodiments of the invention will be afforded to those skilled in the art, as well as a realization of additional advantages thereof, by a consideration of the following detailed description of one or more embodiments. Reference will be made to the appended sheets of drawings that will first be described briefly.

### BRIEF DESCRIPTION OF THE DRAWINGS

Figure 1 is a schematic illustration of a fall detection system, in accordance with one or more embodiments described herein.
Figure 2 is a schematic illustration of a personal module, in accordance with one or more embodiments described herein.
Figure 3 is a schematic illustration of a reference module, in accordance with one or more embodiments described herein.
Figure 4 shows a schematic example of a probability distribution curve for personal pressure data.
Figure 5 schematically shows an example of a house or an apartment where a fall detection system comprising a number of reference modules has been installed.
Figure 6 schematically shows a fall detection method, in accordance with one or more embodiments described herein.

Embodiments of the present disclosure and their advantages are best understood by referring to the detailed description that follows. It should be appreciated that like reference numerals are used to identify like elements illustrated in one or more of the figures.

### DETAILED DESCRIPTION

The present disclosure relates generally to fall detection systems and fall detection methods. Embodiments of the disclosed solution are presented in more detail in connection with the figures.

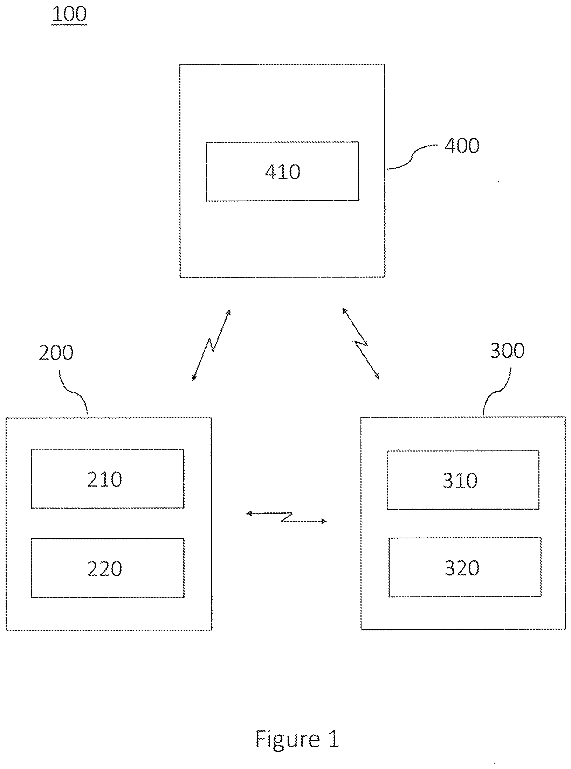

Figure 1 is a schematic illustration of a fall detection system 100, in accordance with one or more embodiments described herein. The fall detection system 100 shown in figure 1 comprises a personal module 200, a reference module 300 and a remote processing arrangement 400, which are arranged to communicate with each other. For clarity, the system 100 is shown comprising only one personal module 200 and only one reference module 300, but the system may comprise any number of personal modules 200 and reference modules 300. In the embodiment shown in figure 1, the personal module 200 comprises a personal module processing device 210 and a personal module pressure sensor 220, the reference module 300 comprises a reference module processing device 310 and a reference module pressure sensor 320, and the remote processing arrangement 400 comprises a remote processing device 410.

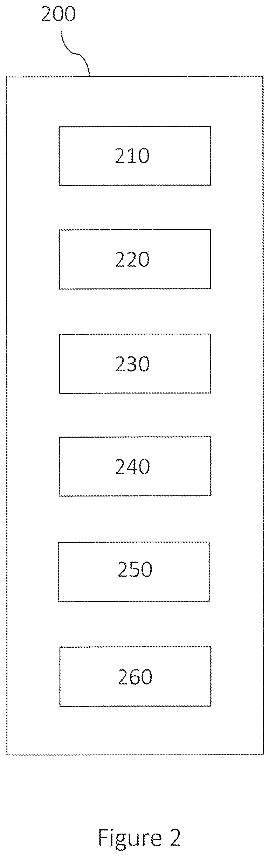

An embodiment of the personal module 200 is shown schematically in figure 2. The personal module 200 may be e.g. a wristband, a bracelet, a smartwatch, a necklace, a pendant, a smartphone, or anything else that can be carried by the individual to be monitored. The personal module 200 may comprise other sensors in addition to the personal module pressure sensor 220, such as e.g. a movement sensor 230 (e.g. an accelerometer) and/or a heart rate sensor 240, and/or other functionalities such as a GPS receiver 250. The personal module 200 may also include e.g. a microphone, a speaker, and/or a so called panic button. The personal module 200 may also include a battery.

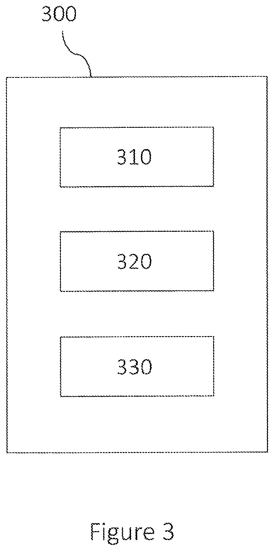

An embodiment of the reference module 300 is shown schematically in figure 3. The reference module 300 may be e.g. a unit that is mounted on the wall or in the ceiling. The reference module 300 may comprise a battery, or be supplied with electricity through being connected with the electrical system in the house or apartment.

The personal module pressure sensor 220 continuously senses personal pressure data that varies with the height of the personal module pressure sensor 220 as the individual moves. The personal pressure data from the personal module pressure sensor 220 is monitored, e.g. by the personal module processing device 210, in order to determine whether a fall has occurred. The personal pressure data may e.g. be in the form of a personal pressure signal from the personal module pressure sensor 220 that is sampled by the personal module processing device 210. In embodiments, the personal pressure data comprises the derivative of the personal pressure signal from the personal module pressure sensor 220, in order for the rate of change to be determined.

There are a number of factors that may cause rapid pressure changes that are of greater magnitude than the pressure change caused by a fall. The ambient pressure may change rapidly due to e.g. weather change, and the pressure in a room may change rapidly due to e.g. the opening or closing of a door or a window. The magnitude of such pressure changes may be greater than the pressure change caused by a fall (e.g. in the form of a drop of 0.5 m). In order to compensate for this, reference pressure data from the reference module pressure sensor 320 may be used together with the personal pressure data from the personal module pressure sensor 220 in the determination of a personal pressure Pp. The personal pressure Pp may e.g. be the same as the personal pressure data from the personal module pressure sensor 220 as long as the reference pressure data from the reference module pressure sensor 320 is considered to be "normal", e.g. by not changing too rapidly or deviating too much from nominal reference pressure values. When the reference pressure data from the reference module pressure sensor 320 is not considered to be "normal", the personal pressure Pp may e.g. be disregarded. It may be enough to make this determination only if the personal pressure P_{P} lies beyond the individual personal pressure profile, in order to prevent erroneous fall detection alerts.

Another option is to determine the personal pressure Pp as an absolute personal pressure P_{A} calculated based on the personal pressure data from the personal module pressure sensor 220 and reference pressure data from the reference module pressure sensor 320. Since the reference pressure data will vary with the pressure changes in the room, calculating an absolute personal pressure P_{A} based on the personal pressure data and the reference pressure data will eliminate any personal pressure changes due to pressure changes in the room. It may be enough to calculate the absolute personal pressure P_{A} only if the personal pressure Pp lies beyond the individual personal pressure profile, in order to prevent erroneous fall detection alerts. The reference pressure data may e.g. be in the form of a reference pressure signal from the reference module pressure sensor 320 that is sampled by the reference module processing device 310. In embodiments, the reference pressure data comprises the derivative of the reference pressure signal from the reference module pressure sensor 320, in order for the rate of change to be determined.

The personal pressure P_{P} is compared with an individual personal pressure profile, which may e.g. be in the form of an individual personal pressure threshold P_{T}, and a fall detection alert is set only if the personal pressure Pp lies beyond the individual personal pressure profile, e.g. by being above the individual personal pressure threshold P_{T}. The setting of a fall detection alert may e.g. involve changing the value of a fall detection parameter or flag. The individual personal pressure profile is determined based on the historical personal pressure Pp for the individual, that may e.g. be stored in a memory. Since the individual personal pressure profile is individually determined, variations in movement pattern between different individuals and between different houses or apartments will be compensated, and the number of false alarms will be reduced.

The fall detection alerts may be used for various purposes. The obvious purpose is to send an alarm signal to call the attention of people who may help the individual who has fallen, such as relatives and/or healthcare personnel. Such an alarm signal may be sent e.g. to a mobile device such as a smartphone, or a multipurpose healthcare alarm system. The system may comprise different levels of alarms based e.g. on the certainty of the fall detection, so that high level alarms are immediately sent out to all relevant parties while low level alarms are routed differently.

The individual who has fallen may not always require help, but it may still be useful to collect information about detected falls. Individuals who fall often may e.g. have health related problems which relatives and/or healthcare personnel may wish to investigate further. Information about detected falls may therefore be stored in a memory for further analysis.

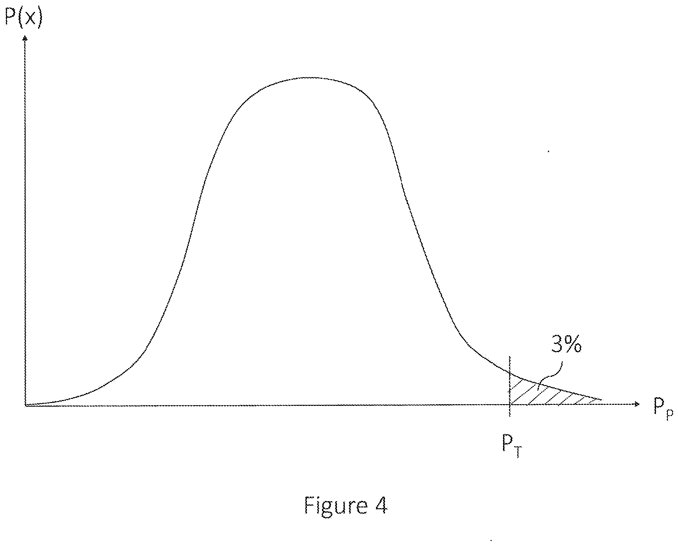

Figure 4 shows a schematic example of a probability distribution curve for the personal pressure Pp of an individual. When enough personal pressure data has been collected for the individual, the personal pressure Pp may be analyzed and arranged using a probability distribution curve, e.g. using histogram functionality. It can then be determined how often the personal pressure Pp lies within various pressure ranges. The individual personal pressure profile used in a fall detection may then be based on the probability distribution of the historical personal pressure Pp for the individual, by e.g. determining a threshold P_{T} in such a way that personal pressure values Pp that are higher than the threshold P_{T} have less than a predetermined probability of occurring. This is a simple yet reliable way of determining the individual personal pressure profile.

The predetermined probability is preferably set to avoid too many false alarms, e.g. at 1-5% probability of occurring, such as at e.g. 3% probability of occurring. This is in figure 4 illustrated with a threshold line marking the point where 3% of the probability under the probability distribution curve lies above the threshold P_{T}, and ensures that the system will not set fall detection alerts too often. However, for an individual who falls often, the threshold line should preferably be moved to the left in the curve, such as to a point where e.g. 8% or 10% of the probability under the probability distribution curve lies above the threshold P_{T}, to ensure that all falls are detected.

In the schematic example of a probability distribution curve for the personal pressure Pp shown in figure 4, the threshold P_{T} has been set to discriminate only personal pressures Pp that are higher than the threshold P_{T}. If the threshold P_{T} is set just to discriminate personal pressures Pp having a low probability of occurring, also unusually low personal pressures P_{P} will be detected. This is usually less relevant, since unusually low personal pressures Pp occur when the personal module is located higher up than it usually is, such as e.g. when the individual is raising the arm. This is normally not associated with a fall. It is thus advantageous to discriminate only personal pressures Pp that are higher than the predetermined threshold P_{T}.

The individual personal pressure profile may be in the form of an individual personal pressure threshold P_{T}, but it may also take other forms. If large amounts of historical personal pressure Pp values for an individual are fed into a machine learning system together with information about e.g. historical personal pressure Pp values indicating a fall, the machine learning system may determine an individual personal pressure profile which is more complex than an individual personal pressure threshold P_{T}, but which may still be used to determine whether a specific personal pressure Pp indicates a fall. Such a machine learning algorithm may in this case also use other data than the historical personal pressure Pp values for the individual in order to determine the individual personal pressure profile. The individual personal pressure profile may e.g. be in the form of a range or pattern of "normal" personal pressure Pp values, and may include also "normal" rates of change of the personal pressure Pp, e.g. based on the derivative of the personal pressure signal from the personal module pressure sensor 220. The comparison of the personal pressure Pp with the individual personal pressure profile may involve comparing the personal pressure Pp over a certain time span with a pattern of "normal" personal pressure Pp values.

A machine learning system may also be trained using historical personal pressures representing detected falls for many individuals. In this case, the fall detection alert may be set based on the comparison of the personal pressure P_{P} with the individual personal pressure profile, together with whether the machine learning system classifies the personal pressure P_{P} as a fall based on historical personal pressures representing detected falls for many individuals. For this comparison, the personal pressure Pp may first be normalized, e.g. by deducting the average and dividing by the standard deviation.

If the personal module 200 comprises additional sensors in addition to the personal module pressure sensor 220, such as e.g. a movement sensor 230 (e.g. an accelerometer) and/or a heart rate sensor 240, the fall detection alert may be based also on signals from these sensors. A fall may comprise a characteristic movement, e.g. in the form of a characteristic sequence of motion and postures. If a large amount of data is analyzed, e.g. using machine learning, e.g. in a so called self-learning system, typical signal patterns corresponding to a fall may be determined. These signal patterns may be detected by a movement sensor, which means that the signal from a movement sensor can be used to avoid false alarms. In the same way, the analysis of a signal from a heart rate sensor 240 can also be used to avoid false alarms. If e.g. the heart rate does not change although the personal pressure Pp indicates a fall, it is likely that a fall has not occurred.

The fall detection system 100 may thus comprise at least one processing device 210, 310, 410, at least one personal module 200 comprising a personal module pressure sensor 220, and at least one reference module 300 comprising a reference module pressure sensor 320. The at least one processing device 210, 310, 410 may be arranged to receive personal pressure data from the at least one personal module pressure sensor 220, receive reference pressure data from the at least one reference module pressure sensor 320, determine a personal pressure Pp using the personal pressure data and the reference pressure data, determine an individual personal pressure profile, which may e.g. be an individual personal pressure threshold P_{T}, based on the historical personal pressure P_{P} for the individual, compare the personal pressure P_{P} with the individual personal pressure profile, and set a fall detection alert based at least on whether the personal pressure Pp lies beyond the individual personal pressure profile, e.g. by being above the individual personal pressure threshold P_{T}. Since the individual personal pressure profile is individually determined, variations in movement pattern between different individuals and between different houses or apartments will be compensated, and the number of false alarms will be reduced.

It is not necessary for the system 100 to comprise any remote processing arrangement 400 - it is perfectly possible for all the processing to take place in the personal module processing device 210 and/or in the reference module processing device 310. In the same way, it is not necessary for the personal module 200 to comprise a personal module processing device 210, or for the reference module 300 to comprise a reference module processing device 310, if the system comprises a remote processing arrangement 400 and all the processing takes place in the remote processing device 410. The system thus only needs to comprise one processing device 210, 310, 410, but may also comprise several processing devices 210, 310, 410 working together.

The personal module 200 may comprise a personal module communication interface 260, and the reference module 300 may comprise a reference module communication interface 330, which are arranged to communicate with each other. This enables communication between the personal module 200 and the reference module 300, so that a personal pressure Pp can be determined using personal pressure data form the personal module pressure sensor 220 and reference pressure data from the reference module pressure sensor 320. In order for this determination to take place in the personal module processing device 210, the reference pressure data must first be received from the reference module pressure sensor 320 in the personal module processing device 210, and this requires a communication interface between the personal module 200 and the reference module 300. In order for this determination to take place in the reference module processing device 310, the reference pressure data must first be received from the personal module pressure sensor 220 in the reference module processing device 310, and this also requires a communication interface between the personal module 200 and the reference module 300. However, this determination may also take place in a remote processing device 410, which may e.g. be comprised in a web server, and in such a case a communication interface to the remote processing arrangement 400 is required, either directly from each of the personal module 200 and the reference module 300, or e.g. from just one of them, if there is a communication interface between them. The communication between all of the units in the system may thus take place either directly or indirectly, via other units. For example, if each of the personal module 200 and the reference module 300 has a communication interface with a remote processing arrangement 400, they may communicate with each other via the remote processing arrangement 400.

The personal module 200 may comprise additional functionalities, such as e.g. a GPS receiver 250. This may be used to determine the location of the personal module 200. The fall detection alert may then be based also on input from the GPS receiver 250. It may e.g. be determined through analysis of historical data in which parts of the house or apartment that a fall is most likely to occur, and this may be used as input into the setting of the fall detection alert. Input from the GPS receiver 250 may also be used to discriminate a person walking down a staircase from a person falling, e.g. based on the speed of downwards movement. Further, input from the GPS receiver 250 may be used to set the severity of the fall detection alert, so that if it is e.g. determined that a fall has occurred in an area containing a staircase, the fall detection alert is set as severe, and alarms are immediately sent out.

The location of the personal module 200 may also be determined in other ways than using a GPS receiver 250, such as e.g. based on from which reference modules 300 signals are received.

Additional sensors 230, 240, 250 in the personal module 200 may also be used to set the severity of the fall detection alert, which may be used to adapt the level of alarms sent out. If the signals from all sensors indicate a fall, the fall detection alert may be set to be severe and high level alarms be immediately sent out to all relevant parties, but if the personal pressure Pp indicates a fall but all other sensors are normal, the fall detection alert may be set to be less severe and just indications be sent to various designated systems. The additional sensors 230, 240, 250 may e.g. be a movement sensor (e.g. an accelerometer), a heart rate sensor, a GPS receiver, or sensors for blood pressure and/or galvanic skin response. The time during which the personal pressure Pp has been beyond the individual personal pressure profile may also be determined. Information from many different sensors may be combined and analyzed, e.g. using machine learning or artificial intelligence, e.g. in order to set the severity of the fall detection alert.

To ensure correspondence between pressure readings from a personal module pressure sensor 220 and a reference module pressure sensor 320, a reference module 300 may be installed in each room that the individual wearing the personal module 200 has access to. The personal pressure data is in this case preferably compared with reference pressure data from the reference pressure sensor 320 that is located in the same room as the individual carrying the personal module 200. Further, the pressure may in some situations also vary within a room due to e.g. size and architecture, and in such cases it may be desirable to install multiple reference modules 300 in a room. The personal pressure data is in this case preferably compared with reference pressure data from the reference pressure sensor 320 that is closest to the individual carrying the personal module 200. Since the reference pressure data should correspond as closely as possible to the personal pressure data, the reference pressure data should be collected as simultaneously as possible from the reference module 300 that is closest to the personal module 200 when the personal pressure data is collected. The determination of which reference module 300 that is closest may e.g. be done in the personal module processing device 210 using e.g triangulation of signals from several reference modules 300, in any of the processing devices 210, 310, 410 using the GPS receiver 250 of the personal module 200 and known locations of the reference modules 300, or by analyzing the relative strength of signals received from the reference modules 300 in the personal module 200. The determination may include pairing the personal module 200 with the reference module 300 that is closest to the personal module 200 in ways known in the art.

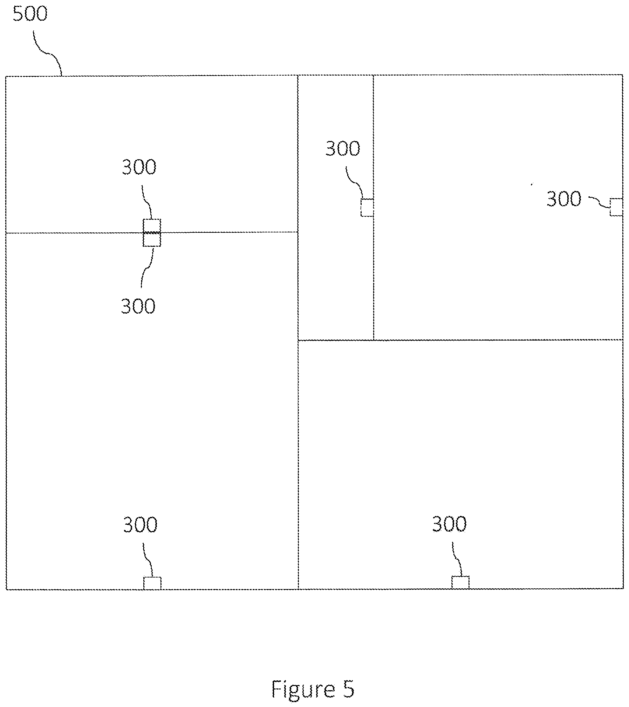

Figure 5 schematically shows an example of a house or an apartment 500 where a fall detection system 100 comprising a number of reference modules 300 has been installed. In the example shown, each room comprises at least one reference module 300, and the largest room comprises two reference modules 300. It is of course not necessary for each room to comprise a reference module 300, although this increases the sensitivity and reliability of the fall detection system 100. The reference modules 300 may be installed in any suitable position, such as e.g. mounted on the walls and/or in the ceiling. If the house or apartment 500 comprises more than one level or floor, there should be reference modules 300 installed on each level or floor. If an individual carrying the personal module 200 moves between the floors, e.g. down a staircase, the system should compensate for this, so that fall detection alerts are not set each time the individual walks down the staircase. This may e.g. be done by determining the position of the personal module 200, e.g. using a GPS receiver 250. Alternatively, the speed of change of the personal pressure Pp may be used, e.g. by analyzing the derivative of the personal pressure P_{P}.

Any appropriate algorithm may be used for the setting of a fall alert based on the combination of the determination of whether the personal pressure Pp lies above the individual personal pressure profile with data from other sensors.

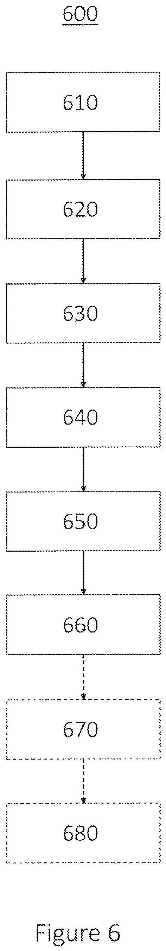

Figure 6 schematically shows a fall detection method 600, in accordance with one or more embodiments described herein. The fall detection method 600 may include the following steps (not necessarily in this order):
Step 610: receiving, in at least one processing device 210, 310, 410, personal pressure data from a personal module pressure sensor 220 arranged in a personal module 200.

Step 620: receiving, in the at least one processing device 210, 310, 410, reference pressure data from a reference module pressure sensor 320 arranged in a reference module 300.

Step 630: determining a personal pressure Pp using the personal pressure data and the reference pressure data.

Step 640: determining an individual personal pressure profile based on the historical personal pressure Pp for the individual.

Step 650: comparing the personal pressure P_{P} with the individual personal pressure profile; and
Step 660: setting a fall detection alert based at least on whether the personal pressure Pp lies beyond the individual personal pressure profile.

If there is more than one reference module 300 in the system, the determining 630 of the personal pressure Pp may use reference pressure data from the reference module 300 that is closest to the personal module 200.

The individual personal pressure profile may comprise an individual personal pressure threshold P_{T}, and the setting 660 of the fall detection alert may in this case be based at least on whether the personal pressure Pp is above the individual personal pressure threshold P_{T}.

The determining 630 of the personal pressure Pp may e.g comprise calculating the personal pressure Pp as an absolute personal pressure P_{A} based on the personal pressure data and the reference pressure data, and the determining 640 of the individual personal pressure profile may e.g. be based on the historical absolute personal pressure P_{A} for the individual.

The personal pressure Pp may e.g. be determined 630 using reference pressure data from the reference module 300 that is closest to the personal module 200.

The fall detection method 600 may further include:
Step 670: inputting the personal pressure Pp into a machine learning system that has been trained using historical personal pressures representing detected falls, wherein the setting 660 of the fall detection alert is based also on whether said machine learning system classifies the personal pressure Pp as a fall.

The machine learning system may be trained using historical personal pressures representing detected falls for the individual, or for many individuals. If the machine learning system has been trained using historical personal pressures representing detected falls for many individuals, the fall detection alert may be set based on the comparison of the personal pressure P_{P} with the individual personal pressure profile, together with whether this machine learning system classifies the personal pressure Pp as a fall based on historical personal pressures representing detected falls for many individuals. For this comparison, the personal pressure Pp may first be normalized, e.g. by deducting the average and dividing by the standard deviation.

The determining 640 of the individual personal pressure profile may e.g. be based on the historical personal pressure Pp of the individual, e.g. based on the probability distribution of the historical personal pressure Pp of the individual, e.g. in such a way that personal pressure values Pp that lie beyond the individual personal pressure profile have less than a predetermined probability of occurring. The predetermined probability of occurring may e.g. be 1-5%, such as e.g. 3%.The setting 660 of the fall detection alert may be based also on a signal from a movement sensor 230, e.g. an accelerometer, arranged in the personal module 200. The setting 660 of the fall detection alert may be based also on other parameters, such as the signal from a heart rate sensor 240 or a GPS receiver 250.

Since the personal pressure profile is individually determined, the claimed method compensates for variations in movement pattern between different individuals and between different houses or apartments, and thus reduces the number of false alarms.

The fall detection method 600 may further comprise:
Step 680: sending an alarm signal based on the fall detection alert.

The fall detection method 600 may further include communicating between the personal module 200 and the reference module 300 using a personal module communication interface 260 and a reference module communication interface 330.

The foregoing disclosure is not intended to limit the present invention to the precise forms or particular fields of use disclosed. It is contemplated that various alternate embodiments and/or modifications to the present invention, whether explicitly described or implied herein, are possible in light of the disclosure. Accordingly, the scope of the invention is defined only by the claims.

## Claims

1. Fall detection system (100) comprising at least one processing device (210, 310, 410), at least one personal module (200) comprising a personal module pressure sensor (220), and at least one reference module (300) comprising a reference module pressure sensor (320), wherein the at least one processing device (210, 310, 410) is arranged to:
receive personal pressure data from the at least one personal module pressure sensor (220);
receive reference pressure data from the at least one reference module pressure sensor (320);
determine a personal pressure (P_{P}) using the personal pressure data and the reference pressure data;
determine an individual personal pressure profile based on the historical personal pressure (P_{P}) of the individual;
compare the personal pressure (P_{P}) with the determined individual personal pressure profile; and
set a fall detection alert based at least on whether the personal pressure (Pp) lies beyond the individual personal pressure profile.

2. Fall detection system (100) according to claim 1, wherein the individual personal pressure profile comprises an individual personal pressure threshold (P_{T}), and the at least one processing device (210, 310, 410) is arranged to set the fall detection alert based at least on whether the personal pressure (P_{P}) is above the individual personal pressure threshold (P_{T}).

3. Fall detection system (100) according to claim 1 or 2, wherein the at least one processing device (210, 310, 410) is further arranged to:
determine the personal pressure (P_{P}) as an absolute personal pressure (P_{A}) calculated based on the personal pressure data and the reference pressure data; and
determine the individually determined personal pressure profile based on the historical absolute personal pressure (P_{A}) of the individual.

4. Fall detection system (100) according to any one of claims 1-3, wherein the at least one processing device (210, 310, 410) is arranged to determine the individual personal pressure profile based on the probability distribution of the historical personal pressure (P_{P}) of the individual.

5. Fall detection system (100) according to any one of the preceding claims, wherein the personal module (200) further comprises a movement sensor (230), e.g. an accelerometer, and the at least one processing device (210, 310, 410) is further arranged to analyze the signal from the movement sensor (230) and set the fall detection alert based also on this signal.

6. Fall detection system (100) according to any one of the preceding claims, wherein the processing device (210, 310, 410) is further arranged to send an alarm signal based on the fall detection alert.

7. Fall detection system (100) according to any one of the preceding claims, comprising a number of reference modules (300), wherein the at least one processing device (210, 310, 410) is arranged to determine the personal pressure (P_{P}) using reference pressure data from the reference module (300) that is closest to the personal module (200).

8. Fall detection method (600), comprising:
receiving (610), in at least one processing device (210, 310, 410), personal pressure data from a personal module pressure sensor (220) arranged in a personal module (200);
receiving (620), in the at least one processing device (210, 310, 410), reference pressure data from a reference module pressure sensor (320) arranged in a reference module (300);
determining (630) a personal pressure (P_{P}) using the personal pressure data and the reference pressure data;
determining (640) an individual personal pressure profile based on the historical personal pressure (P_{P}) of the individual;
comparing (650) the personal pressure (P_{P}) with the individual personal pressure profile; and
setting (660) a fall detection alert based at least on whether the personal pressure (P_{P}) lies beyond the individual personal pressure profile.

9. Fall detection method (600) according to claim 8, wherein the individual personal pressure profile comprises an individual personal pressure threshold (P_{T}), and the setting (660) of the fall detection alert is based at least on whether the personal pressure (Pp) is above the individual personal pressure threshold (P_{T}).

10. Fall detection method (600) according to claim 8 or 9, wherein the determining (630) of the personal pressure (P_{P}) comprises calculating the personal pressure (P_{P}) as an absolute personal pressure (P_{A}) based on the personal pressure data and the reference pressure data, and the determining (640) of the individually determined personal pressure profile is based on the historical absolute personal pressure (P_{A}) of the individual.

11. Fall detection method (600) according to any one of claims 8-10, wherein the determining (640) of the individual personal pressure profile is based on the probability distribution of the historical personal pressure (P_{P}) of the individual.

12. Fall detection method (600) according to any one of claims 8-11, further comprising inputting (670) the personal pressure (P_{P}) into a machine learning system that has been trained using historical personal pressures representing detected falls, wherein the setting (660) of the fall detection alert is based also on whether said machine learning system classifies the personal pressure (P_{P}) as a fall.

13. Fall detection method (600) according to any one of claims 8-12, wherein the setting (660) of the fall detection alert is based also on a signal from a movement sensor (230), e.g. an accelerometer, arranged in the personal module (200).

14. Fall detection method (600) according to any one of claims 8-13, further comprising sending (680) an alarm signal based on the fall detection alert.

15. Fall detection method (600) according to any one of claims 8-14, wherein the determining (630) of the personal pressure (P_{P}) uses reference pressure data from the reference module (300) that is closest to the personal module (200).

## Patentansprüche

1. Sturzerkennungssystem (100), umfassend wenigstens eine Verarbeitungsvorrichtung (210, 310, 410), wenigstens ein Personenmodul (200), umfassend einen Personenmodul-Drucksensor (220) und wenigstens ein Referenzmodul (300), umfassend einen Referenzmodul-Drucksensor (320), wobei die wenigstens eine Verarbeitungsvorrichtung (210, 310, 410) angeordnet ist, um:
Personendruckdaten von dem wenigstens einen Personenmodul-Drucksensor (220) zu empfangen;
Referenzdruckdaten von dem wenigstens einen Referenzmodul-Drucksensor (320) zu empfangen;
einen Personendruck (Pp) unter Verwendung der Personendruckdaten und der Referenzdruckdaten zu bestimmen;
ein individuelles Personendruckprofil basierend auf dem historischen Personendruck (Pp) der Einzelperson zu bestimmen;
den Personendruck (Pp) mit dem bestimmten individuellen Personendruckprofil zu vergleichen; und
einen Sturzerkennungsalarm einzustellen, der wenigstens darauf basiert, ob der Personendruck (Pp) außerhalb des individuellen Personendruckprofils liegt.

2. Sturzerkennungssystem (100) nach Anspruch 1, wobei das individuelle Personendruckprofil einen individuellen Personendruckschwellenwert (P_{T}) umfasst, und die wenigstens eine Verarbeitungsvorrichtung (210, 310, 410) angeordnet ist, um den Sturzerkennungsalarm wenigstens basierend darauf einzustellen, ob der Personendruck (Pp) über dem individuellen Personendruckschwellenwert (P_{T}) liegt.

3. Sturzerkennungssystem (100) nach Anspruch 1 oder 2, wobei die wenigstens eine Verarbeitungsvorrichtung (210, 310, 410) ferner angeordnet ist, um:
den Personendruck (P_{P}) als einen absoluten Personendruck (P_{A}) zu bestimmen, der basierend auf den Personendruckdaten und den Referenzdruckdaten berechnet wird; und
Bestimmen des individuell bestimmten Personendruckprofils basierend auf dem historischen absoluten Personendruck (P_{A}) der Einzelperson.

4. Sturzerkennungssystem (100) nach einem der Ansprüche 1-3, wobei die wenigstens eine Verarbeitungsvorrichtung (210, 310, 410) angeordnet ist, um das individuelle Personendruckprofil basierend auf der Wahrscheinlichkeitsverteilung des historischen Personendrucks (Pp) der Einzelperson zu bestimmen.

5. Sturzerkennungssystem (100) nach einem der vorhergehenden Ansprüche, wobei das Personenmodul (200) ferner einen Bewegungssensor (230), z.B. einen Beschleunigungsmesser, umfasst und die wenigstens eine Verarbeitungsvorrichtung (210, 310, 410) ferner angeordnet ist, um das Signal von dem Bewegungssensor (230) zu analysieren und den Sturzerkennungsalarm auch basierend auf diesem Signal einzustellen.

6. Sturzerkennungssystem (100) nach einem der vorangehenden Ansprüche, wobei die Verarbeitungsvorrichtung (210, 310, 410) ferner angeordnet ist, ein Alarmsignal basierend auf dem Sturzerkennungsalarm zu senden.

7. Sturzerkennungssystem (100) nach einem der vorhergehenden Ansprüche, umfassend eine Anzahl von Referenzmodulen (300), wobei die wenigstens eine Verarbeitungsvorrichtung (210, 310, 410) angeordnet ist, um den Personendruck (Pp) unter Verwendung von Referenzdruckdaten von dem Referenzmodul (300) zu bestimmen, das dem Personenmodul (200) am nächsten ist.

8. Sturzerkennungsverfahren (600), umfassend:
Empfangen (610) von Personendruckdaten von einem Personenmodul-Drucksensor (220), der in einem Personenmodul (200) angeordnet ist, in wenigstens einer Verarbeitungsvorrichtung (210, 310, 410);
Empfangen (620) von Referenzdruckdaten von einem Referenzmodul-Drucksensor (320), der in einem Referenzmodul (300) angeordnet ist, in der wenigstens einen Verarbeitungsvorrichtung (210, 310, 410);
Bestimmen (630) eines Personendrucks (Pp) unter Verwendung der Personendruckdaten und der Referenzdruckdaten;
Bestimmen (640) eines individuellen Personendruckprofils basierend auf dem historischen Personendruck (Pp) der Einzelperson;
Vergleichen (650) des Personendrucks (Pp) mit dem individuellen Personendruckprofil; und
Einstellen (660) eines Sturzerkennungsalarms, der wenigstens darauf basiert, ob der Personendruck (Pp) außerhalb des individuellen Personendruckprofils liegt.

9. Sturzerkennungsverfahren (600) nach Anspruch 8, wobei das individuelle Personendruckprofil einen individuellen Personendruckschwellenwert (P_{T}) umfasst, und das Einstellen (660) des Sturzerkennungsalarms wenigstens darauf basiert, ob der Personendruck (Pp) über dem individuellen Personendruckschwellenwert (P_{T}) liegt.

10. Sturzerkennungsverfahren (600) nach Anspruch 8 oder 9, wobei das Bestimmen (630) des Personendrucks (P_{P}) Berechnen des Personendrucks (P_{P}) als einen absoluten Personendruck (P_{A}) basierend auf den Personendruckdaten und den Referenzdruckdaten umfasst, und das Bestimmen (640) des individuell bestimmten Personendruckprofils auf dem historischen absoluten Personendruck (P_{A}) der Einzelperson basiert.

11. Sturzerkennungsverfahren (600) nach einem der Ansprüche 8-10, wobei das Bestimmen (640) des individuellen Personendruckprofils auf der Wahrscheinlichkeitsverteilung des historischen Personendrucks (Pp) der Einzelperson basiert.

12. Sturzerkennungsverfahren (600) nach einem der Ansprüche 8-11, ferner umfassend Eingeben (670) des Personendrucks (Pp) in ein maschinelles Lernsystem, das unter Verwendung historischer Personendrücke, die erfasste Stürze repräsentieren, trainiert wurde, wobei das Einstellen (660) des Sturzerkennungsalarms auch darauf basiert, ob das maschinelle Lernsystem den Personendruck (Pp) als einen Sturz klassifiziert.

13. Sturzerkennungsverfahren (600) nach einem der Ansprüche 8 bis 12, wobei das Einstellen (660) des Sturzerkennungsalarms auch auf einem Signal von einem Bewegungssensor (230), z.B. einem Beschleunigungsmesser, der in dem Personenmodul (200) angeordnet ist, basiert.

14. Sturzerkennungsverfahren (600) nach einem der Ansprüche 8-13, ferner umfassend Senden (680) eines Alarmsignals basierend auf dem Sturzerkennungsalarm.

15. Sturzerkennungsverfahren (600) nach einem der Ansprüche 8-14, wobei das Bestimmen (630) des Personendrucks (Pp) Referenzdruckdaten von dem Referenzmodul (300) verwendet, das dem Personenmodul (200) am nächsten ist.

## Revendications

1. Système (100) de détection de chute comprenant au moins un dispositif de traitement (210, 310, 410), au moins un module personnel (200) comprenant un capteur de pression (220) de module personnel, et au moins un module de référence (300) comprenant un capteur de pression (320) de module de référence, ledit au moins un dispositif de traitement (210, 310, 410) étant agencé de façon à :
recevoir des données de pression personnelle en provenance dudit au moins un capteur de pression (220) de module personnel ;
recevoir des données de pression de référence en provenance dudit au moins un capteur de pression (320) de module de référence ;
déterminer une pression personnelle (P_{P}) en utilisant les données de pression personnelle et des données de pression de référence ;
déterminer un profil de pression personnel d'individu sur la base de la pression personnelle historique (P_{P}) de l'individu ;
comparer la pression personnelle (P_{P}) avec le profil de pression personnel d'individu déterminé ; et
définir une alerte de détection de chute basée au moins sur le fait que la pression personnelle (P_{P}) se situe au-delà du profil de pression personnel d'individu.

2. Système (100) de détection de chute selon la revendication 1, dans lequel le profil de pression personnelle d'individu comprend un seuil de pression personnelle d'individu (P_{T}), et ledit au moins un dispositif de traitement (210, 310, 410) est agencé pour définir l'alerte de détection de chute. sur la base au moins du fait que la pression personnelle (P_{P}) est supérieure au seuil de pression personnelle d'individu (P_{T}).

3. Système (100) de détection de chute selon la revendication 1 ou la revendication 2, dans lequel ledit au moins un dispositif de traitement (210, 310, 410) est en outre agencé pour :
déterminer la pression personnelle (P_{P}) en tant que pression personnelle absolue (P_{A}) calculée sur la base des données de pression personnelle et des données de pression de référence ; et
déterminer le profil de pression personnel déterminé individuellement sur la base de la pression personnelle absolue (P_{A}) historique de l'individu.

4. Système (100) de détection de chute selon l'une quelconque des revendications 1 à 3, dans lequel ledit au moins un dispositif de traitement (210, 310, 410) est agencé pour déterminer le profil de pression personnel d'individu sur la base de la distribution de probabilité de la pression personnelle (P_{P}) historique de l'individu.

5. Système (100) de détection de chute selon l'une quelconque des revendications précédentes, dans lequel le module personnel (200) comprend en outre un capteur de mouvement (230), par ex. un accéléromètre, et ledit au moins un dispositif de traitement (210, 310, 410) est en outre agencé de façon à analyser le signal provenant du capteur de mouvement (230) et à définir l'alerte de détection de chute sur la base également de ce signal.

6. Système (100) de détection de chute selon l'une quelconque des revendications précédentes, dans lequel le dispositif de traitement (210, 310, 410) est en outre agencé pour envoyer un signal d'alarme basé sur l'alerte de détection de chute.

7. Système (100) de détection de chute selon l'une quelconque des revendications précédentes, comprenant plusieurs modules de référence (300), ledit au moins un dispositif de traitement (210, 310, 410) étant agencé pour déterminer la pression personnelle (P_{P}) en utilisant des données de pression de référence provenant du module de référence (300) qui est le plus proche du module personnel (200).

8. Procédé (600) de détection de chute, comprenant :
le fait (610) de recevoir, dans au moins un dispositif de traitement (210, 310, 410), des données personnelles de pression en provenance d'un capteur de pression (220) de module personnel agencé dans un module personnel (200) ;
le fait (620) de recevoir, dans ledit au moins un dispositif de traitement (210, 310, 410), des données de pression de référence en provenance d'un capteur de pression (320) de module de référence agencé dans un module de référence (300) ;
le fait (630) de déterminer une pression personnelle (P_{P}) en utilisant les données de pression personnelle et des données de pression de référence ;
le fait (640) de déterminer un profil de pression personnel d'individu en utilisant la pression personnelle historique (P_{P}) de l'individu ;
le fait (650) de comparer la pression personnelle (P_{P}) avec le profil de pression personnelle d'individu ; et
le fait (660) de définir une alerte de détection de chute basée au moins sur le fait que la pression personnelle (P_{P}) se situe au-delà du profil de pression personnelle d'individu.

9. Procédé (600) de détection de chute selon la revendication 8, dans lequel le profil de pression personnelle d'individu comprend un seuil de pression personnel d'individu (P_{T}), et la définition (660) de l'alerte de détection de chute est basée au moins sur le fait que la pression personnel (P_{P}) est supérieure au seuil de pression personnel (P_{T}) d'individu.

10. Procédé (600) de détection de chute selon la revendication 8 ou la revendication 9, dans lequel la détermination (630) de la pression personnelle (P_{P}) comprend le fait de calculer la pression personnelle (P_{P}) en tant que pression personnelle absolue (P_{A}) sur la base des données de pression personnelle et des données de pression de référence, et la détermination (640) du profil de pression personnelle déterminé individuellement est basée sur la pression personnelle absolue (P_{A}) historique de l'individu.

11. Procédé (600) de détection de chute selon l'une quelconque des revendications 8 à 10, dans lequel la détermination (640) du profil de pression personnelle d'individu est basée sur la distribution de probabilité de la pression personnelle (P_{P}) historique de l'individu.

12. Procédé (600) de détection de chute selon l'une quelconque des revendications 8 à 11, comprenant en outre le fait (670) de saisir la pression personnelle (P_{P}) dans un système d'apprentissage automatique qui a fait l'objet d'un apprentissage en utilisant des pressions personnelles historiques représentant des chutes détectées, la définition (660) de l'alerte de détection de chute étant également basé sur le fait que ledit système d'apprentissage automatique classe la pression personnelle (P_{P}) comme étant une chute.

13. Procédé (600) de détection de chute selon l'une quelconque des revendications 8 à 12, dans lequel la définition (660) de l'alerte de détection de chute est également basé sur un signal provenant d'un capteur de mouvement (230), par ex. un accéléromètre, agencé dans le module personnel (200).

14. Procédé (600) de détection de chute selon l'une quelconque des revendications 8 à 13, comprenant en outre le fait (680) d'envoyer un signal d'alarme basé sur l'alerte de détection de chute.

15. Procédé (600) de détection de chute selon l'une quelconque des revendications 8 à 14, dans lequel la détermination (630) de la pression personnelle (P_{P}) utilise des données de pression de référence provenant du module de référence (300) qui est le plus proche du module personnel (200).
